# EUROPEAN PATENT SPECIFICATION

(11) **EP 1 523 366 B1**
(45) Date of publication and mention of the grant of the patent: **05.09.2012**
(21) Application number: 02750291.3
(22) Date of filing: 23.07.2002
(51) Int. Cl.: A61M 25/01

(54) **GUIDEWIRE WITH TAPERED FLEXIBLE CORE SEGMENT**
FÜHRUNGSDRAHT MIT EINEM KONISCHEN, FLEXIBLEN KERNSEGMENT
FIL-GUIDE AVEC SEGMENT CENTRAL SOUPLE EFFILE

(43) Date of publication of application: 20.04.2005
(73) Proprietor: Abbott Cardiovascular Systems Inc., Santa Clara, CA 95054 (US)
(72) Inventor: CORNISH, Wayne, E., Fallbrook, CA 92028 (US); RICHARDSON, Mark, T., Escondido, CA 92026 (US); BRENNAN, Lawrence, Temecula, CA 92592 (US); JALISI, Marc, M., Santa Clara, CA 95054-2807 (US); ANDERSON, David, M., Temecula, CA 92592 (US); JAFARI, Mo, Murrieta, CA 92591 (US); FARIABI, Sepehr, Santa Clara, CA 95054-2807 (US)
(74) Representative: McLeish, Nicholas Alistair Maxwell
(86) International application number: PCT/US2002/023544
(87) International publication number: WO 2004/009170

(56) References cited:
- EP-A- 0 495 299
- WO-A-00/40288
- US-A- 4 003 369
- US-A- 5 788 654
- US-B1- 6 491 648

## Description

### BACKGROUND OF THE INVENTION

This invention relates to the field of guidewires for advancing intraluminal devices such as stent delivery catheters, balloon dilatation catheters, atherectomy catheters and the like within body lumens.

In a typical percutaneous coronary procedure, a guiding catheter having a pre-formed distal tip is percutaneously, introduced into a patient's peripheral artery, e.g. femoral or brachial artery, by means of a conventional Seldinger technique and advanced therein until the distale tip of the guiding catheter is seated in the ostium of a desired coronary artery. A guidewire is first advanced by itself through the guiding catheter until the distal tip of the guidewire extends beyond the arterial location where the procedure is to be performed. Then a rapid exchange type catheter, such as described in U.S. Patent No. 5,061,395 (Yock) is mounted onto the proximal portion of the guidewire which extends out of the proximal end of the guiding catheter which is outside of the patient. The catheter is advanced over the guidewire, while the position of the guidewire is fixed, until the operative element on the rapid exchange type catheter is disposed within the arterial location where the procedure is to be performed. After the procedure is performed, the rapid exchange type catheter may be withdrawn from the patient over the guidewire or the guidewire repositioned within the coronary anatomy for an additional procedure.

A guidewire may also be used in conjunction with the delivery of an intracoronary stent. One method and system involves disposing a compressed or otherwise small diameter stent about an expandable member such as a balloon on the distal end of a catheter, advancing the catheter through the patient's vascular system over a guidewire until the stent is in the desired location within a blood vessel. The expendable member on the catheter may then be expanded to expand the stent within the blood vessel. The dilated expandable member is then contracted and the catheter withdrawn, leaving the expanded stent within the blood vessel, holding the passageway thereof open. This latter method and system can be used concurrently with balloon angioplasty or subsequent thereto.

Further details of guidewires, and devices associated therewith for various interventional procedures can be found in U.S. Patent No. 4,748,986 (Morrison et al.); U.S. Patent No. 4,538,622 (Samson et al.); U.S. Patent No. 5,135,503 (Abrams); U.S. Patent No. 5,341,818 (Abrams et al.); and U.S. Patent No. 5,345,945 (Hodgson, et al.) .

Conventional guidewires for angioplasty, stent delivery, atherectomy and other intravascular procedures usually have an elongate core member with one or more segments near the distal end thereof which taper distally to smaller cross sections. A flexible body member, such as a helical coil or a tubular body of polymeric material, is typically disposed about and secured to at least part of the distal portion of the core member. A flexible core segment, which may be the distal extremity of the core member or a separate shapeable ribbon which is secured to the distal extremity of the core member extends through the flexible body and is secured to the distal end of the flexible body by soldering, brazing or welding, or an adhesive in the case of polymeric flexible bodies which forms a rounded distal tip. The leading tip is highly flexible and will not damage or perforate the vessel and the portion behind the distal tip is increasingly stiff which better supports a balloon catheter or similar device.

The flexible core segment or ribbon of a typical guidewire is a small diameter wire which has been flattened to a relatively constant transverse profile. Flattening of the flexible core segment facilitates the shapability of the member. However, a flexible core segment having a constant transverse profile or flexibility can be subject to prolapse during use. Prolapse occurs when the flexible core segment gets bent back on itself in a constrained lumen and is difficult to straighten out with proximal manipulation.

Against this background there is described in EP-A-0,495,299 a guidewire for use in guiding a catheter which has a tip construction that includes a core wire within a helical coil. The core wire has a distal tip segment that is tapered and is further impressed with a pair of opposed flattened surfaces extending along the taper. The surfaces lie at an angle to each other and define a progressively flattened tip having increased width in a distal direction to define a duckbill configuration. The tip construction is said to provide improved column strength at the tip to resist guidewire prolapse while providing adequate torsional and flexibility characteristics.

### SUMMARY OF THE INVENTION

According to the present invention there is provided a guidewire having the features of claim 1.

The present invention is directed to a guidewire having an elongate core member with a proximal core section and a distal core section, and a flexible body disposed about and secured to at least a portion of the distal core section. The guidewire has an elongated, preferably shapeable flexible core segment which is secured to or which is formed as part of the distal core section and which is secured to the distal end of the flexible body disposed about the distal core section. The distal core section may have one or more tapered sections proximal to the flexible core segment which have distally decreasing tapers with substantially round transverse cross sections.

The tapered, preferably shapeable flexible core segment has a double reverse taper, i.e. a first transverse dimension which distally tapers over a substantival length thereof from a first value to a second smaller value and a second transverse dimension which distally tapers over essentially the same length of the flexible core segment from a first value to a second larger value, i.e. flares outwardly. The length of the tapered flexible core segment is about 1 to about 12 cm, preferably about 2 to about 10 cm. At least 50%, preferably at least 75% of the length of the tapered flexible core segment is tapered as described above. The distal most portion of the flexible core segment (i.e. up to about 15 mm) may be flat with one or both of the opposed faces being parallel.

The flexible core segment has two pairs of opposing faces which are essentially the mirror image of each other. In one of the pairs the opposing faces converge toward each other while in the other pair the opposing faces diverge from each other.

The flexible core segment may be formed integrally or out of the distal extremity of the distal core section or may be formed as a distinct structural component or shaping ribbon which needs to be mounted in a suitable manner to the distal core section, e.g. by welding, brazing, soldering, adhesive bonding, mechanical connections and other known mounting processes. The flexible core segment may by formed from round or flattened wire and may be coined or rolled or otherwise plastically deformed, e.g. cold forged, to a desired shape and sectional profile. When the flexible core segment is a separate member from the core member, it may be formed before or after it is secured to the core member.

The flexible body member is disposed about the flexible core segment, preferably along its entire length and may take the form of a helical coil, polymer jacket, or the like. The distal end of the flexible body member is secured to the distal end of the flexible core segment and an intermediate portion of the flexible body member is preferably secured to the distal core section proximal to the tapered flexible core segment

The double taper of the flexible core segments on the distal parts of the guidewire reduces the likelihood of prolapsing or kinking of the guidewire's distal extremity during procedures and may be used to provide a controlled longitudinal variation and transition in flexibility of the core segment to the distal tip of the guidewire. A flexible body member, such as a helical coil or a tubular plastic member, having a proximal end and a distal end is typically disposed about and secured to the distal section of the elongate core member.

The taper geometry of the flexible segment may be modeled mathematically. Specific taper or face contours may be selected in keeping with the principles of the invention to achieve optimum performance for specific usage requirements. These and other advantages of the invention will become more apparent from the following detailed description of the invention when taken in conjunction with the accompanying exemplary drawings.

### BRIEF DESCRIPTION OF THE DRAWINGS

FIG. 1 is an elevation view, partially in section, of a guidewire embodying features of the invention.
FIG. 2 is an enlarged elevational view of a portion of the distal core section of the guidewire shown in FIG. 1.
FIG. 3 is an enlarged elevational view of a portion of the distal core section of the guidewire shown in FIG. 1 taken 90° from the view shown in FIG. 2
FIG. 4 is an end view of the embodiment of FIG. 3, shown as viewed from line 4-4 in FIG. 3.
FIG. 5 is an elevational view of an alternative core member for a guidewire such as shown in FIG. 1 having separate flexible core segment having features of the invention, mounted onto the distal extremity of a guidewire core member.
FIG. 6 is an elevational view of another alternative core member similar to that shown in FIG. 5 but which has been shaped after the flexible core segment is secured to the core member.

### DETAILED DESCRIPTION OF THE INVENTION

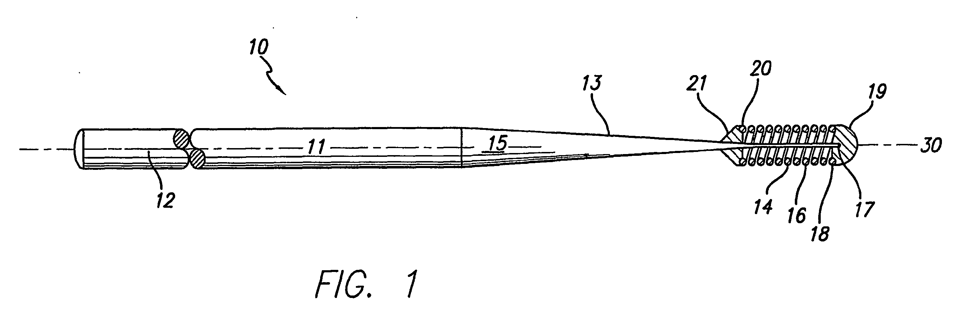

FIG. 1 is an elevational view of an embodiment of a guidewire 10 which embodies features of the invention, and which includes an elongated core member 11 with a proximal core section 12, a distal core section 13, and a flexible body member or coil 14 which is disposed about and fixed to the distal core section 13. The distal core section 13 has a tapered core segment 15 and a flexible core segment 16 which is distally contiguous to the tapered core segment 15 and a distal end 17 which is secured to the distal end 18 of the coil 14 by a rounded body of solder or weld 19. The proximal end 20 of the coil 14 is similarly bonded or secured to the distal core section 13 by a body of solder 21.

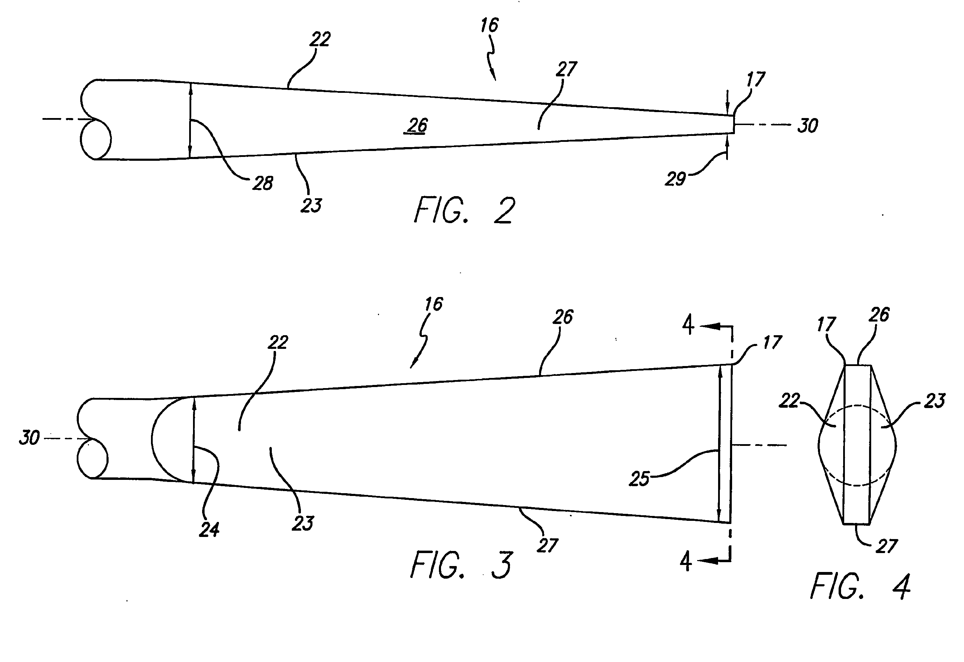

The flexible core segment 16, which is shown in more detail in Figs. 2-5, has a first pair of opposed faces 22 and 23 which taper distally from a first transverse dimension 24 to a larger second transverse dimension 25 and a second pair of opposed faces 26 and 27 which taper distally from a first transverse dimension 28 to a smaller transverse dimension 29. The first transverse dimensions 24 and 28 of the first and second pairs of opposed faces respectively may have same value or different values. Thus, flexible core segment 16 tapers to become progressively thinner in one transverse direction and wider in a second transverse direction as the distal end 17 is approached. This results in a smooth decrease in stiffness in one direction but a smooth increase in stiffness in a second direction perpendicular to the first direction. While each of the tapered faces 22,23,26,27 is shown as having a longitudinal contour that is substantially straight, the second pair of opposed tapered faces 26,27 have a curved longitudinal contour as may also at least one of the first pair of opposed tapered faces 22,23. The side face 22 is shown generally as a mirror image of the side face 23 about the longitudinal axis 30 and the top face 26 is shown generally as a mirror image of the bottom face 27 about the axis 30. However, the opposed faces need not be symmetrical.

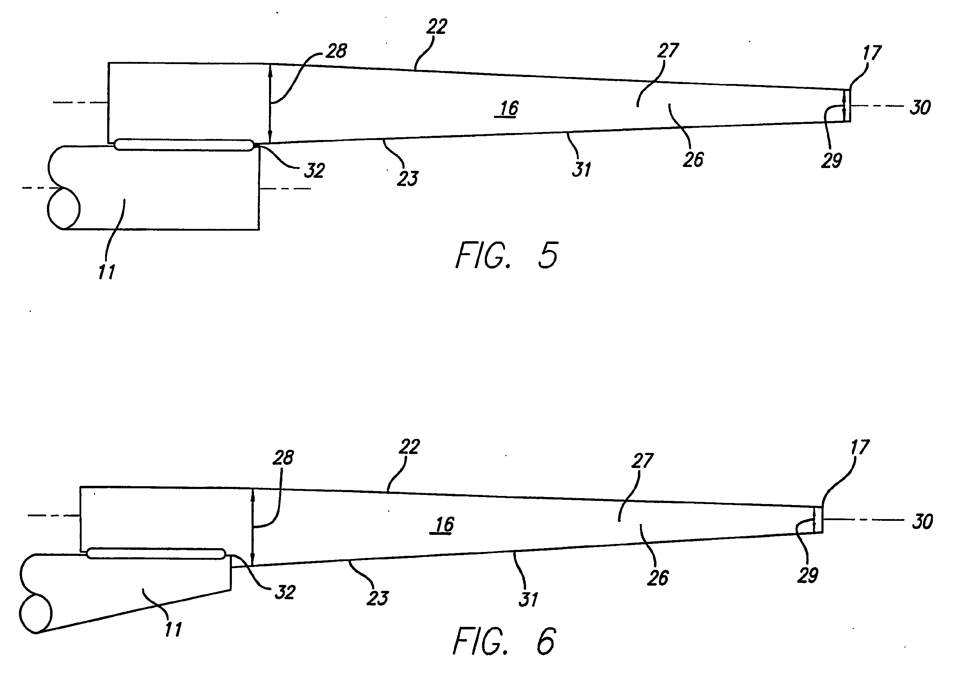

In Figs. 1-4, the flexible core segment 16 is shown as being an integral part of the distal core section 13. In Fig. 5 and 6, the flexible core segment 16 is shown as a separate shaping member 31 which has been secured to the distal end of the core member 11 by solder or weldment 32. The opposing pairs of faces are the same as that shown in Figs. 2-4 and are provided with the same reference numbers. In Fig. 5 the shaping member 31 is shaped before being secured to the core member 11, whereas, in Fig. 6 the shaping member 31 and the distal tip of the core member 11 has been shaped after being joined together.

The flexible segment 16 has a length typically ranging about 1 to about 12 cm, preferably about 2 to about 10 cm, although longer segments may be used. At least 50% and preferably at least 75% of the length of the flexible segment 16 is tapered. The form of taper of the flexible segment 16 provides a controlled longitudinal variation and transition in flexibility of the distal core section. The first transverse or thickness dimension of the taper at a proximal portion of the flexible segment 16 is about 0.025mm to about 0.09mm (0.001-0.0035 inch), preferably about 0.04mm to about 0.06mm (0.0015-0.0025 inch) which tapers to about 0.01 mm to about 0.06mm (0.0005-0.0025 inch), preferably about 0.02mm to about 0.04mm (0.0007-0.0015 inch) at the distal portion. The second transverse dimension or width, perpendicular to the first transverse dimension, of the taper at a proximal portion of the flexible segment 16 is about 0.025mm to about 0.09mm (0.001-0.0035 inch), preferably about 0.04mm to about 0.06mm (0.0015-0.0025 inch) which flares to about 0.05mm to about 0.2mm (0.002-0.008 inch), preferably about 0.08mm to about 0.15mm (0.003-0.006 inch).

The multiple tapers or faces of the flexible core segment 16 are preferably formed by impact forging or rolling a wire or ribbon of suitable dimensions, but other methods may be employed.

Thus, it may be seen in FIGS. 2 and 3 that the thickness of the flexible core segment 16 decreases distally as measured between faces 22 and 23, while the width of the flexible segment 16 increases distally as measured between side faces 26 and 27. The cross section of the flexible core segment may be generally rectangular in all or in a portion of its length. While not shown, a transition may be provided which tapers from the round transverse cross-section of the proximal portion of the flexible tapered section 16 to the rectangular cross-section of the tapered portion; so as to create a smooth transition at the proximal end of the flexible segment 16.

The flexible core segment may vary in length from about 0.25 cm to about 10 cm, although both shorter and longer segments may be desirable in some procedures. The width and thickness at each cross sectional portion may be varied to provide the desired stiffness along the distal portion of the guidewire.

The distal section 13 may also have more than one tapered segment 15 which have typical distally decreasing tapers with substantially round transverse cross sections, such as describes, in U.S. Patent Application Ser. No. 081868,764, filed June 4, 1997 (Cornish, et al.) entitled STEERABLE GUIDEWIRE WITH ENHANCED DISTAL SUPPORT.

The core member 11 may be formed of stainless steel, NiTi alloys or combinations thereof such as described in U.S. Patent No. 5,341,818 (Abrams et al). Other materials such as the high ——————————————————————— strength alloys described in U.S. Patent No. 5,636,641 (Fariabi), entitled HIGH STRENGTH MEMBER FOR INTRACORPOREAL USE, ___________ __________ may also be used. The core member 11 may be optionally coated with a lubricious coating such as a fluoropolymer, e.g. TEFLON ® available from DuPont, which extends at least the length of the proximal core section 12. The distal section 13 is also provided with a lubricous coating, such as a MICROGLIDE ™ coating (a silicone material). Hydrophilic coatings may also be employed.

The overall length and diameter of guidewire 10 may be varied to suit the particular procedures in which it is to be used and the materials from which it is constructed. The length of the guidewire 10 generally ranges from about 65 cm to about 320 cm, more typically ranging from about 160 cm to about 200 cm. Commercially available guidewires for coronary anatomy, typically have lengths of about 175 cm or about 190 cm for the coronary anatomy. Guidewire diameters generally range from about 0.2mm to about 0.9mm (0.008 to 0.035 inch), more typically ranging from about 0.25mm to about 0.55mm (0.01 to 0.018 inch).

Commercially available guidewires for coronary use are typically about 0.25, 0.3 and 0.036mm (0.01, 0.012 and 0.014 inch) in diameter.

The wire from which the coil 14 is made generally has a transverse diameter of about 0.025mm to about 0.1mm (0.001-0.004 inch), preferably about 0.05mm to about 0.008mm (0.002-0.003 inch). Multiple turns of the distal portion of — coil 14 may be expanded to provide additional flexibility. The helical coil 14 may have a diameter or transverse dimension that is about the same as the proximal core section 12. The coil 14 may have a length of about 2 to about 40 cm or more, preferably about 2 to about 10 cm in length. The coil 14 may at least in part be formed of a suitable radiopaque material such as platinum, palladium or alloys thereof or formed of other material such as stainless steel and coated with a radiopaque material such as gold. The coil 14 may be replaced with a flexible body member formed of a polymeric material such as polyimide, polyethylene, polyurethane, polytetrafluoroethylene (PTFE), expanded polytetrafluoroethylene (ePTFE) and other similar materials.

While particular forms of the invention have been illustrated and described, it will be apparent that various modifications can be made without departing from the scope of the invention. Accordingly, it is not intended that the invention be limited, except as by the appended claims. Moreover, those skilled in the art will recognize that a feature found in one embodiment may be utilized in another embodiment.

## Claims

1. A guidewire (10) for advancing intracorporeal devices to a desired location within a patient's body, comprising:
a. an elongate core member (11) with a proximal core section (12) and a distal core section (13) with an elongated flexible core segment (16) which has a first transverse dimension tapering distally from a first value (28) to a second smaller value (29) and which has a second transverse dimension tapering distally from a first value (24) to a second larger value (25);
b. the elongated flexible core segment (16) includes a first pair of opposed tapered faces (22,23) which define the first transverse dimension and a second air of opposed tapered faces (26,27) which define the second transverse dimension (24) the second pair of opposed tapered faces (26,27) having a longitudinal contour that is curved; and
c. a flexible body (14) disposed about and secured to at least a portion of the distal core section (13).

2. The guidewire (10) of claim 1 wherein the elongated flexible core segment (16) is a member separate from the core member (11) and which has a distal end (17) secured to the distal end (18) of the flexible body (14) disposed about the distal core section (13) and a proximal end secured to (32) the core member (11).

3. The guidewire (10) of claim 1 wherein at least one of the first pair of opposed faces (22,23) has a longitudinal contour that is substantially straight.

4. The guidewire (10) of claim 1 wherein at least one of the first pair of opposed faces (22,23) has a longitudinal contour that is curved.

5. The guidewire (10) of claim1 wherein the first pair of opposed faces (22,23) have a transverse dimension greater than a corresponding transverse dimension of the second pair of opposed faces (26,27) along a substantial length of the flexible core segment (16).

6. The guidewire (10) of claim 1 wherein the first pair of opposed faces (22,23) is substantially normal to the second pair of opposed faces (26,27).

7. The guidewire (10) of claim 1 wherein the elongated flexible core segment (16) has a length of about 1 to about 12cm.

8. The guidewire (10) of claim 1 wherein the elongated flexible core segment (16) has a length of about 2 to about 10cm.

9. The guidewire (10) of claim 1 wherein at least 50% of the length of the flexible core segment (16) is tapered.

10. The guidewire (10) of claim 1 wherein at least 75% of the length of the flexible core segment (16) is tapered.

11. The guidewire (10) of claim 1 wherein the taper of the flexible core segment (16) is configured to provide a controlled longitudinal variation and transition in flexibility.

12. The guidewire (10) of claim 1 wherein the first transverse dimension of the taper at a proximal portion of the flexible core segment (16) is about 0.025mm to about 0.09mm (0.001-0.0035 inch).

13. The guidewire (10) of claim 1 wherein the first transverse dimension of the taper at the proximal portion of the flexible core segment (16) is about 0.04mm to about 0.06mm (0.0015-0.0025 inch).

14. The guidewire (10) of claim 14 wherein the first transverse dimension tapers to about 0.01mm to about 0.06mm (0.0005-0.0025 inch).

15. The guidewire (10) of claim 16 wherein the first transverse dimension tapers to about 0.02mm to about 0.04mm (0.0007 - 0.015 inch).

16. The guidewire (10) of claim 1 wherein the second transverse dimension of the taper, perpendicular to the first transverse dimension, at a proximal portion of the flexible core segment (16) is about 0.025mm to about 0.09mm (0.001-0.0035 inch).

17. The guidewire (10) of claim 1 wherein the second transverse dimension of the taper, perpendicular to the first transverse dimension, at a proximal portion of the flexible core segment (16) is about 0.04mm to about 0.006mm (0.0015-0.0025 inch).

18. The guidewire (10) of claim 19 wherein the second transverse dimension of the flexible core segment (16) flares to about 0.05mm to about 0.2mm (0.002 - 0.008 inch).

19. The guidewire (10) of claim 19 wherein the second transverse dimension of the flexible core segment (16) flares to about 0.08mm to about 0.15mm (0.003-0.006 inch).

## Patentansprüche

1. Führungsdraht (10) zum Befördern von intrakorporalen Vorrichtungen zu einer gewünschten Position innerhalb des Körpers eines Patienten, welcher Führungsdraht folgendes aufweist:
a. ein längliches Kernelement (11) mit einem proximalen Kernabschnitt (12) und einem distalen Kernabschnitt (13) mit einem länglichen flexiblen Kernsegment (16), das eine erste transversale Abmessung, die sich distal von einem ersten Wert (28) zu einem zweiten kleineren Wert (29) verjüngt, aufweist und das eine zweite transversale Abmessung, die sich distal von einem ersten Wert (24) zu einem zweiten größeren Wert (25) verjüngt, aufweist;
b. das längliche flexible Kernsegment (16), das ein erstes Paar an gegenüberliegenden konischen Flächen (22, 23) aufweist, die die erste transversale Abmessung definieren, und ein zweites Paar an gegenüberliegenden konischen Flächen (26, 27) aufweist, die die zweite transversale Abmessung (24) definieren, wobei das zweite Paar an gegenüberliegenden konischen Flächen (26, 27) eine Längskontur aufweist, die gebogen ist; und
c. einen flexiblen Körper (14), der um mindestens ein Teil des distalen Kernabschnitts (13) herum angeordnet und an diesem befestigt ist.

2. Führungsdraht (10) nach Anspruch 1, wobei das längliche flexible Kernsegment (16) ein von dem Kernelement (11) getrenntes Element ist und das ein distales Ende (17) aufweist, das an dem distalen Ende (18) des flexiblen Körpers (14) befestigt ist, der um den distalen Kernabschnitt (13) herum angeordnet ist, und ein proximales Ende, das an dem Kernelement (11) befestigt ist.

3. Führungsdraht (10) nach Anspruch 1, wobei mindestens eine Fläche des ersten Paares an gegenüberliegenden Flächen (22, 23) eine Längskontur aufweist, die im Wesentlichen gerade ist.

4. Führungsdraht (10) nach Anspruch 1, wobei mindestens eine Fläche des ersten Paares an gegenüberliegenden Flächen (22, 23) eine Längskontur aufweist, die gebogen ist.

5. Führungsdraht (10) nach Anspruch 1, wobei das erste Paar an gegenüberliegenden Flächen (22, 23) eine transversale Abmessung aufweist, die größer ist als eine entsprechende transversale Abmessung des zweiten Paares an gegenüberliegenden Flächen (26, 27) entlang einer wesentlichen Länge des flexiblen Kernsegments (16).

6. Führungsdraht (10) nach Anspruch 1, wobei das erste Paar an gegenüberliegenden Flächen (22, 23) im Wesentlichen senkrecht zu dem zweiten Paar an gegenüberliegenden Flächen (26, 27) ist.

7. Führungsdraht (10) nach Anspruch 1, wobei das längliche flexible Kernsegment (16) eine Länge von ungefähr 1 bis ungefähr 12 cm aufweist.

8. Führungsdraht (10) nach Anspruch 1, wobei das längliche flexible Kernsegment (16) eine Länge von ungefähr 2 bis ungefähr 10 cm aufweist.

9. Führungsdraht (10) nach Anspruch 1, wobei mindestens 50% der Länge des flexiblen Kernsegments (16) konisch ist.

10. Führungsdraht (10) nach Anspruch 1, wobei mindestens 75% der Länge des flexiblen Kernsegments (16) konisch ist.

11. Führungsdraht (10) nach Anspruch 1, wobei die Verjüngung des flexiblen Kernsegments (16) so ausgestaltet ist, dass sie eine kontrollierte längslaufende Veränderung und Überleitung der Flexibilität aufweist.

12. Führungsdraht (10) nach Anspruch 1, wobei die erste transversale Abmessung der Verjüngung an einem proximalen Abschnitt des flexiblen Kernsegments (16) ungefähr 0,025mm bis ungefähr ungefähr 0,09mm (0,001 - 0,0035 Zoll) ist.

13. Führungsdraht (10) nach Anspruch 1, wobei die erste transversale Abmessung der Verjüngung an dem proximalen Abschnitt des flexiblen Kernsegments (16) ungefähr 0,04mm bis ungefähr 0,06mm (0,0015 - 0,0025 Zoll) ist.

14. Führungsdraht (10) nach Anspruch 12, wobei sich die erste transversale Abmessung auf ungefähr 0,01 mm bis ungefähr 0,06mm (0,0005 - 0,0025 Zoll) verjüngt.

15. Führungsdraht (10) nach Anspruch 14, wobei sich die erste transversale Abmessung auf ungefähr 0,02mm bis ungefähr 0,04mm (0,0007 - 0,015 Zoll) verjüngt.

16. Führungsdraht (10) nach Anspruch 1, wobei die zweite transversale Abmessung der Verjüngung, lotrecht zu der ersten transversalen Abmessung, an einem proximalen Abschnitt des flexiblen Kernsegments (16) ungefähr 0,025mm bis ungefähr 0,09mm (0,001 - 0,0035 Zoll) ist.

17. Führungsdraht (10) nach Anspruch 1, wobei die zweite transversale Abmessung der Verjüngung, lotrecht zu der ersten transversalen Abmessung, an einem proximalen Abschnitt des flexiblen Kernsegments (16) ungefähr 0,04mm bis ungefähr 0,006mm (0,0015 - 0,0025 Zoll) ist.

18. Führungsdraht (10) nach Anspruch 17, wobei sich die zweite transversale Abmessung des flexiblen Kernsegments (16) auf ungefähr 0,05mm bis ungefähr 0,2mm (0,002 - 0,008 Zoll) aufweitet.

19. Führungsdraht (10) nach Anspruch 17, wobei sich die zweite transversale Abmessung des flexiblen Kernsegments (16) auf ungefähr 0,08mm bis ungefähr 0,15mm (0,003 - 0,006 Zoll) aufweitet.

## Revendications

1. Fil-guide (10) pour faire avancer des dispositifs intracorporels à un emplacement souhaité dans le corps d'un patient, comprenant:
a. un élément d'âme oblong (11) avec une section d'âme proximale (12) et une section d'âme distale (13) avec un segment d'âme flexible oblong (16) qui a une première dimension transversale diminuant distalement d'une première valeur (28) à une deuxième valeur plus petite (29) et qui a une deuxième dimension transversale diminuant distalement d'une première valeur (24) vers une deuxième valeur plus grande (25);
b. le segment d'âme flexible oblong (16) comprend une première paire de faces diminuées opposées (22, 23) qui définissent la première dimension transversale, et une deuxième paire de faces diminuées opposées (26, 27) qui définissent la deuxième dimension transversale (24), la deuxième paire de faces diminuées opposées (26, 27) ayant un contour longitudinal qui est courbé; et
c. un corps flexible (14) disposé autour et fixé à au moins une portion de la section d'âme distale (13).

2. Fil-guide (10) selon la revendication 1, dans lequel le segment d'âme flexible oblong (16) est un élément séparé de l'élément d'âme (11), et qui possède une extrémité distale (17) fixée à l'extrémité distale (18) du corps flexible (14) disposé autour de la section d'âme distale (13) et une extrémité proximale fixée (32) à l'élément d'âme (11).

3. Fil-guide (10) selon la revendication 1, dans lequel au moins une de la première paire de faces opposées (22, 23) possède un contour longitudinal qui est sensiblement rectiligne.

4. Fil-guide (10) selon la revendication 1, dans lequel au moins une de la première paire de faces opposées (22, 23) possède un contour longitudinal qui est courbé.

5. Fil-guide (10) selon la revendication 1, dans lequel la première paire de faces opposées (22, 23) ont une dimension transversale plus grande qu'une dimension transversale correspondante de la deuxième paire de faces opposées (26, 27) sur une longueur substantielle du segment d'âme flexible (16).

6. Fil-guide (10) selon la revendication 1, dans lequel la première paire de faces opposées (22, 23) est sensiblement normale à la deuxième paire de faces opposées (26, 27).

7. Fil-guide (10) selon la revendication 1, dans lequel le segment d'âme flexible oblong (16) a une longueur d'environ 1 à environ 12 cm.

8. Fil-guide (10) selon la revendication 1, dans lequel le segment d'âme flexible oblong (16) a une longueur d'environ 2 à environ 10 cm.

9. Fil-guide (10) selon la revendication 1, dans lequel au moins 50% de la longueur du segment d'âme flexible (16) sont diminués.

10. Fil-guide (10) selon la revendication 1, dans lequel au moins 75% de la longueur du segment d'âme flexible (16) sont diminués.

11. Fil-guide (10) selon la revendication 1, dans lequel la diminution du segment d'âme flexible (16) est configurée pour permettre une variation longitudinale contrôlée et une transition en flexibilité.

12. Fil-guide (10) selon la revendication 1, dans lequel la première dimension transversale de la diminution à la portion proximale du segment d'âme flexible (16) est d'environ 0,025 mm à environ à 0,09 mm (0,001-0,0035 pouce).

13. Fil-guide (10) selon la revendication 1, dans lequel la première dimension transversale de la diminution à la portion proximale du segment d'âme flexible (16) est d'environ 0,04 mm à environ 0,06 mm (0,0015-0,0025 pouce).

14. Fil-guide (10) selon la revendication 12, dans lequel la première dimension transversale diminue à environ 0,01 mm jusqu'à environ 0,06 mm (0,0005-0,0025 pouce).

15. Fil-guide (10) selon la revendication 14, dans lequel la première dimension transversale diminue à environ 0,02 mm jusqu'à environ 0,04 mm (0,0007-0,015 pouce).

16. Fil-guide (10) selon la revendication 1, dans lequel la deuxième dimension transversale de la diminution, perpendiculaire à la première dimension transversale, à une portion proximale du segment d'âme flexible (16) est d'environ 0,025 mm à environ 0,09 mm (0,001-0,0035 pouce).

17. Fil-guide (10) selon la revendication 1, dans lequel la deuxième dimension transversale de la diminution, perpendiculaire à la première dimension transversale, à une portion proximale du segment d'âme flexible (16) est d'environ 0,04 mm à environ 0,006 mm (0,0015-0,0025 pouce).

18. Fil-guide (10) selon la revendication 17, dans lequel la deuxième dimension transversale du segment d'âme flexible (16) s'élargit à environ 0,05 mm jusqu'à environ 0,2 mm (0,002-0,008 pouce).

19. Fil-guide (10) selon la revendication 17, dans lequel la deuxième dimension transversale du segment d'âme flexible (16) s'élargit à environ 0,08mm jusqu'à environ 0,15 mm (0,003-0,006 pouce).
